# DEMANDE DE BREVET EUROPEEN

(11) **EP 0 834 306 A2**
(43) Date de publication de la demande: **08.04.1998**
(21) Numéro de dépôt: 97402123.0
(22) Date de dépôt: 12.09.1997
(51) Int. Cl.: A61K 7/48, A61K 7/00

(54) **Emulsion cosmétique E/H à forte teneur en électrolytes**

(30) Priorité: 07.10.1996 FR 9612196
(71) Demandeur: LA ROCHE POSAY Laboratoire Pharmaceutique, 86270 La Roche Posay (FR)
(72) Inventeur: Burnier, Véronique, 75012 Paris (FR)
(74) Mandataire: Tezier Herman, Béatrice

(57) **Abrégé**

La présente invention se rapporte à une émulsion eau dans huile stable, à forte teneur en électrolytes, comprenant au moins 2 % en poids par rapport au poids total de la composition d'un sel métallique hydrosoluble, utile notamment pour une application topique, en particulier pour une utilisation en dermo-cosmétique, pour traiter les phénomènes d'irritation et/ou de peaux sensibles.

## Description

La présente invention se rapporte à une émulsion eau dans huile stable, à forte teneur en électrolytes, comprenant au moins 2 % en poids par rapport au poids total de la composition d'un sel métallique hydrosoluble, au moins un gélifiant hydrophobe et un système émulsionnant approprié en quantité suffisante pour obtenir une composition stable, utile notamment pour une application topique. L'invention concerne également l'utilisation de la composition selon l'invention en dermo-cosmétique, notamment pour les désordres pathologiques et/ou physiologiques associés à la libération de la substance P et/ou du TNF-alpha (Tumor Necrosis Factor-alpha) et notamment de traiter les peaux sensibles, les désordres et maladies cutanées où existe un prurit, la rosacée et/ou l'érythème pudique.

Il est connu que certaines peaux sont plus sensibles que d'autres. Les symptômes des peaux sensibles étaient jusqu'à présent mal caractérisés et le problème de ces peaux était, de ce fait, mal défini, personne ne connaissant exactement le processus mis en cause dans la sensibilité -hyperréactivité cutanée non allergique- de la peau. Certains pensaient qu'une peau sensible était une peau qui réagissait aux produits cosmétiques et/ou dermatologiques, d'autres qu'il s'agissait d'une peau qui réagissait à plusieurs facteurs extérieurs, pas forcément liés aux produits cosmétiques et/ou dermatologiques.

Certains tests ont été essayés pour tenter de cerner les peaux sensibles, par exemple des tests à l'acide lactique et au DMSO qui sont connus pour être des substances irritantes : voir par exemple l'article de K. Lammintausta et al., Dermatoses, 1988, 36, pages 45-49; et l'article de T. Agner et J. Serup, Clinical and Experimental Dermatology, 1989, 14, pages 214-217. Mais ces tests ne permettaient pas de caractériser les peaux sensibles, que l'on assimilait à des peaux allergiques.

Les symptômes liés aux peaux sensibles ont été mis en évidence et décrits dans la demande de brevet FR 95 04268 déposée le 10 avril 1995 au nom de L'OREAL. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

Il a été montré en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

Les peaux sensibles pouvaient être scindées en deux grandes formes cliniques, les peaux irritables et les peaux intolérantes. Une peau irritable est une peau qui réagit par un prurit, c'est-à-dire par des démangeaisons ou par des picotements, à différents facteurs tels que l'environnement, les émotions, les aliments, le vent, les frottements, le rasoir, l'eau dure à forte concentration de calcaire, les variations de température ou la laine. En général, ces signes sont associés à une peau sèche avec ou sans dartres, ou à une peau qui présente un érythème. Une peau intolérante est une peau qui réagit par des sensations d'échauffement, de tiraillements, de fourmillements et/ou de rougeurs, à différents facteurs tels que l'application de produits cosmétiques ou dermatologiques ou de savon. En général, ces signes sont associés à un érythème et à une peau hyperséborrhéique ou acnéique, voire même rosacéiforme, avec ou sans dartres.

D'une manière générale, les peaux sensibles se définissent par une réactivité particulière de la peau. Cette hyper-réactivité peut être notamment mise en jeu par des facteurs environnementaux, émotionnels, alimentaires ou encore par l'application ou le contact de produits cosmétiques ou dermatologiques. Cet état d'hyper-réactivité qui définit les peaux sensibles différencie celles-ci de la réactivité ubiquitaire provoquée par des agents irritants qui induisent une irritation de la peau chez la quasi totalité des individus.

Cet état d'hyper-réactivité est ressentie et reconnue par les individus qui en sont atteints comme une "peau sensible".

Les cuirs chevelus "sensibles" ont une sémiologie clinique plus univoque : les sensations de prurit et/ou de picotements et/ou d'échauffements sont essentiellement déclenchées par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à l'eau dure à forte concentration en calcaire et/ou aux variations de température.

Le prurit est un symptôme fréquent des dermatoses, occasionnant une gêne souvent notable pour le patient. lorsque le prurit est très sévère, la gêne peut être telle que le patient ne peut poursuivre son activité habituelle. En outre, le prurit peut être source de complications : excoriations qui peuvent se surinfecter, lichénification des zones prurigineuses, qui a pour conséquence d'installer la maladie dans un véritable cercle vicieux. Parmi les dermatoses fréquemment associées à un prurit, on peut citer l'eczéma, la dermatite atopique, les dermites de contact, les lichens plans, les prurigos, les urticaires, les toxidermies prurigineuses, certaines formes cliniques de psoriasis.

Le prurit est parfois le signe pathologique cutané prédominant comme dans les cas du prurit aquagénique, du prurit du cuir chevelu lors des états pelliculaires *(pityriasis capitis),* du prurit des hémodialysés, des insuffisants rénaux, des sidéens et des personnes atteintes d'obstructions biliaires, ou encore du prurit des manifestations paranéoplastiques de certains cancers.

De plus, le prurit est un signe fréquemment rencontré au cours de certaines affections parasitaires cutanées ou générales. Il peut s'agir par exemple de scabiose, de filariose, d'oxyurose ou encore de démodéciose cutanée.

Du fait que l'on connaissait mal les caractéristiques des peaux sensibles, il était jusqu'à présent très difficile de les traiter, et on les traitait indirectement, par exemple en limitant dans les compositions cosmétiques ou dermatologiques l'emploi de produits à caractère irritant tels que les tensioactifs, les conservateurs ou les parfums ainsi que certains actifs.

Jusqu'à ce jour, les prurits étaient traités à l'aide de préparations émollientes, de corticoïdes locaux, de puvathérapie ou encore d'antihistaminiques. Les corticoïdes locaux sont certes très efficaces pour calmer les symptômes mais malheureusement, leur effet n'est pas immédiat. De plus, ils présentent des effets secondaires souvent très pénalisants comme des atrophies, et exposent à des risques d'infections mycosique et/ou bactérienne. La puvathérapie est l'irradiation locale de la peau malade avec des UVA, après absorption d'une substance photosensibilisante (psoralène). Cette technique présente les inconvénients graves d'un photovieillissement pouvant entraîner des cancers de la peau. De plus, ce traitement n'est pas ambulatoire, obligeant les malades à se rendre couramment dans un centre spécialisé pendant toute la durée du traitement, ce qui est très contraignant et limite leur activité professionnelle. Les émollients ont un effet anti-prurigineux très modeste et sont peu efficaces lorsque les prurits sont importants. Par ailleurs, les antihistaminiques n'ont pas une efficacité constante et nécessitent une prise orale.

Il subsiste donc le besoin d'un traitement de ces affections de la peau, ne présentant pas ces inconvénients.

L'utilisation d'au moins un sel métallique, en particulier d'un métal alcalino-terreux, permettant de traiter efficacement le prurit ou les problèmes de "peau sensible", tout en remédiant aux inconvénients mentionnés ci-dessus a été décrite décrits dans la demande de brevet FR 95 04268 déposée le 10 avril 1995 au nom de L'OREAL.

Des compositions gélifiées à forte teneur en électrolytes sont décrites dans les demandes de brevet FR 96 00742 et FR 96 03094 déposées le 27 janvier 1996 et le 12 mars 1996 au nom de L'OREAL.

La présente invention concerne une nouvelle composition, émulsion eau dans huile (E/H) comprenant une forte teneur en sels métalliques, permettant en particulier de traiter les problèmes de "peau sensible", notamment de prurit. La composition selon l'invention est de préférence une composition cosmétique ou dermatologique.

Dans le domaine cosmétique ou dermatologique, il est courant d'utiliser des crèmes constituées d'une émulsion E/H comportant une phase aqueuse dispersée dans une phase huileuse. Ces émulsions présentent fréquemment des problèmes de stabilité, rendant leur fabrication difficile. Aussi, différents moyens ont été envisagés pour remédier à cet inconvénient. Un moyen d'y remédier consiste à augmenter fortement la teneur en émulsionnant de ces émulsions. Or, on sait que les émulsionnants utilisés en grande quantité peuvent se montrer irritants vis-à-vis de certains types de peau. Par ailleurs, comme précédemment, les crèmes obtenues sont souvent compactes et lourdes. On comprendra aisément que de telles compositions ne sont pas adaptées pour une application sur des peaux sensibles, compte tenu des problèmes évoqués ci-dessus.

Différentes compositions, émulsions E/H à forte teneur en électrolyte sont décrites dans l'état de la technique. Ainsi, la demande de brevet EP-A-530 531 (Benckiser), décrit une composition cosmétique sous forme d'émulsion H/E ou E/H comprenant un système émulsionnant et au moins 2 % (préférentiellement au moins 5 %) d'un sel hydrosoluble de métaux alcalins ou alcalino-terreux. Le sel est de préférence un sel de magnésium qui agit comme conservateur. L'invention objet de cette demande permet d'obtenir des compositions cosmétiques sans conservateurs. Pour les émulsions E/H, le système émulsionnant est constitué par 5 à 10 % en poids de l'association d'un ester de glycérol, d'un copolymères de silicones alkyle polyéthers et d'un ester d'acide gras. De même, le brevet US 5 162 378 (Revlon), décrit une microémulsion E/H comprenant 8 à 20 % d'un sel de métaux alcalins ou alcalino-terreux, 20 à 40 % d'eau et 8 à 20 % d'un système émulsionnant constitué par du cétyl diméthicone copolyol, éventuellement additionné de hexyllaurate et de polyglycéryl-4 isostéarate. Ces compositions comprenant une forte teneur en émulsionnant et une quantité limitée d'eau ne sont pas non plus adaptées à une application sur des peaux sensibles.

Il est donc important de pouvoir disposer d'une composition stable, émulsion E/H, comportant une forte teneur en électrolyte et appropriée pour une application topique, en particulier pour traiter les problèmes de peaux sensibles et de prurit.

La présente invention concerne donc une nouvelle composition sous forme d'émulsion E/H à forte teneur en électrolytes, comprenant au moins 2 % en poids, de préférence au moins 3 % en poids, plus préférentiellement au moins 5 % en poids par rapport au poids total de la composition d'au moins un sel métallique hydrosoluble, au moins un gélifiant hydrophobe et moins de 5 % en poids par rapport au poids total de la composition d'un système émulsionnant approprié.

Par sel métallique, on entend selon la présente invention un sel d'un métal, c'est à dire d'un corps simple susceptible de libérer des cations simples (Dictionnaire de la Chimie et de ses Applications, DUVAL & DUVAL, 3ème Edition, 1978, Technique et Documentation).

Les sels métalliques hydrosolubles sont plus particulièrement choisis parmi les sels hydrosolubles de métaux alcalins, de métaux alcalino-terreux, de métaux de transition et de métaux des groupes 13 et 14 de la classification périodique des éléments.

Comme sels hydrosolubles de métaux alcalins utiles selon l'invention, on peut citer en particulier les sels de lithium, de sodium ou de potassium.

Comme sels hydrosolubles de métaux alcalino-terreux utiles dans l'invention, on peut citer en particulier les sels de béryllium, de magnésium, de calcium, de strontium et/ou de baryum.

Comme sels hydrosolubles de métaux de transition utiles selon l'invention, on peut citer en particulier les sels de lanthanides, et les sels de métaux de la quatrième période de la classification périodique des éléments, comme les sels de magnésium, de cobalt ou de zinc.

Comme sels hydrosolubles de métaux des groupes 13 et 14 de la classification périodique des éléments utiles selon l'invention, on peut citer les sels d'aluminium et d'étain.

Par lanthanide, on entend les éléments de numéro atomique z allant de 57 à 71, c'est-à-dire le lanthane, le cérium, le praséodyme, le néodyme, le prométhium, le samarium, l'europium, le gadolinium, le terbium, le dysprosium, l'holmium, l'erbium, le thulium, l'ytterbium, le lutétium.

De manière préférentielle, les sels métalliques hydrosolubles selon l'invention sont choisis parmi les sels de lithium, de strontium, de baryum, d'yttrium, de néodyme, de gadolinium, de manganèse et de zinc, plus préférentiellement de strontium.

Ces sels peuvent être par exemple des carbonates, des bicarbonates, des sulfates, des glycérophosphates, des borates, des chlorures, des nitrates, des acétates, des hydroxydes, des persulfates ainsi que des sels d'α-hydroxyacides ou des sels d'acides de fruits (citrate, tartrate, lactate, malate), ou encore des sels d'acides aminés (aspartate, arginate, glucocholate, fumarate) ou des sels d'acides gras (palmitate, oléate, caséinate, béhénate).

De préférence le sel est choisi parmi les nitrates ou les chlorures, en particulier le nitrate de lithium, de strontium, de baryum, d'yttrium, de néodyme, de gadolinium, de manganèse ou de zinc, le chlorure de lithium, de strontium, de baryum, d'yttrium, de néodyme, de gadolinium, de manganèse ou de zinc, les sulfates ou les acétates, comme le sulfate de calcium, de strontium ou de magnésium et l'acétate de strontium ou de magnésium.

D'une manière avantageuse, la quantité de sels métalliques hydrosolubles dans la composition selon l'invention est comprise entre 2 % et 20 % en poids par rapport au poids total de la composition, de préférence comprise entre 5 % et 10 % en poids.

Par système émulsionnant approprié, on entend tout agent émulsionnant ou mélange d'agents émulsionnants susceptibles de produire une émulsion E/H à forte teneur en électrolytes stable lorsqu'ils sont présents dans la composition en une teneur inférieure à 5 % en poids par rapport au poids total de la composition.

Par émulsion E/H stable, on entend de préférence selon l'invention une émulsion qui reste stable au moins 1 mois à 45°C.

D'une manière avantageuse, le système émulsionnant approprié est constitué par au moins un ester de glycérol et/ou au moins un polymère siliconé, appropriés pour la préparation d'émulsions E/H.

De préférence, l'ester de glycérol est un ester de polyglycérol et d'acide gras, en particulier un ester de polyglycérol et d'acide (iso)stéarique, tels que les polyglycéryl-n isostéarates, n étant un entier compris entre 2 et 6, commercialisés sous la dénomination ISOLAN par la société GOLDSCHMIDT.

D'une manière avantageuse, le polymère siliconé approprié pour la préparation d'une émulsion E/H est un polysiloxane, de préférence un copolymère de polysiloxane polyalkyle polyéther, tels que ceux décrits dans les demandes de brevet DE-A-36 22 571, EP-A-125 779, EP-A-310 903, EP-A-537 003, ou dans les brevets US-A-4 421 656, US-A-4 218 250, US-A-4 268 499, US-A-4 311 695, US-A-4 122 029, US-A-4 268 499 ou US-A-5 008 103. Il s'agit plus particulièrement de copolymères tels que le cétyl diméthicone copolyol commercialisé sous la dénomination ABIL par la société GOLDSCHMIDT ou le lauryl méthicone copolyol commercialisé sous la dénomination DC Q2-5200 par la société DOW CORNING.

De manière préférentielle, le système émulsionnant approprié est constitué par au moins un polymère siliconé approprié pour la préparation d'émulsions E/H , seul ou en association avec au moins un ester de glycérol.

Lorsqu'ils sont employés en association, le rapport pondéral ester de glycérol / silicone est avantageusement compris entre 1/1 et 1/10, plus préférentiellement d'environ 1/5.

De manière préférentielle, la quantité de système émulsionnant approprié est comprise entre 2 % et 4 % en poids par rapport au poids total de la composition.

En outre, la composition selon l'invention contient au moins un gélifiant hydrophobe. Parmi les gélifiants hydrophobes utiles dans l'émulsion selon l'invention, on peut citer les argiles modifiées comme les bentones, la silice, en particulier la silice hydrophobe, comme la silice silylée ou diméthyl silylée ("Silica Silylate" ou "Silica Dimethyl Silylate") commercialisée sous les dénominations Aerosil R 812, R 972 ou R 974 par la société DEGUSSA, les dérivés d'esters de glycol et d'acide gras, comme le stéarate de glycol acétylé, éventuellement associé à un mono-, di- ou tri-ester de glycérol et d'acide gras, comme la tristéarine, en particulier le mélange stéarate de glycol acétylé / tristéarine commercialisé sous la dénomination Unitiwix par la société GUARDIAN. L'agent gélifiant utilisé est de préférence la silice silylée ou diméthyl silylée ou l'UNITWIX.

La quantité de gélifiant hydrophobe est avantageusement comprise entre 0,01 et 5 % en poids par rapport au poids total de la composition, de préférence comprise entre 0,1 et 3 % en poids.

La phase aqueuse de l'émulsion selon l'invention peut comprendre de l'eau, de l'eau purifiée, une eau florale telle que l'eau de bleuet, ou une eau thermale ou minérale naturelle et leurs mélanges. Par exemple l'eau thermale ou minérale naturelle peut être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades, l'eau de Tercis-les-bains, l'eau d'Avène ou l'eau d'Aix les Bains.

La phase aqueuse est de préférence constituée par une eau thermale connue pour ses propriété apaisantes, anti-irritantes, anti-radicalaires, appliquée sur la peau, en particulier l'eau de la Roche Posay. De telles eaux thermales ont généralement une forte teneur en électrolytes, comprenant notamment des sels hydrosolubles de métaux alcalino-terreux. Bien entendu la quantité de métaux alcalino-terreux dans l'eau thermale sera prise en compte pour déterminer la quantité totale de métaux alcalino-terreux dans la composition selon l'invention.

Ladite phase aqueuse peut être présente à une teneur comprise entre 40 % et 80 % en poids par rapport au poids total de la composition, de préférence comprise entre 50 % et 70 % en poids.

La phase grasse de l'émulsion selon l'invention peut comprendre des corps gras usuellement utilisés dans le domaine d'application envisagé. Parmi ceux-ci, on peut citer les corps gras siliconés tels que les huiles de silicone, ainsi que les corps gras non siliconés tels que les huiles végétales, minérales, animales ou synthétiques.

Parmi les corps gras siliconés, on peut citer :
- (i) les polyalkyl(C₁-C₂₀) siloxanes et notamment ceux à groupements terminaux triméthylsilyle, parmi lesquels on peut citer les polydiméthylsiloxanes linéaires (PDMS) et les alkylméthylpolysiloxanes tels que la cétyldiméthicone (nom CTFA),
- (ii) les huiles de silicone volatiles, telles que :
   - les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
   - les silicones volatiles linéaires ayant de 2 à 9 atomes de silicium. Il s'agit par exemple de l'hexaméthyldisiloxane, de l'hexyl heptaméthyltrisiloxane ou de l'octyl heptaméthyltrisiloxane,

Parmi les corps gras non siliconés, on peut citer les huiles usuelles, telles que l'huile de paraffine, de vaseline, l'huile d'amande, le perhydrosqualène, l'huile d'abricot, l'huile de germes de blé, l'huile d'amande douce, l'huile de callophyllum, l'huile de palme, l'huile de ricin, l'huile d'avocat, l'huile de jojoba, l'huile d'olive ou l'huile de germes de céréales; des esters d'acides gras ou d'alcools gras, tels que l'octyl dodécyl myristate ou les benzoates d'alkyle en C₁₂-C₁₅, des alcools; des acétylglycérides; des octanoates, décanoates ou ricinoléates d'alcools ou de polyalcools; des triglycérides d'acides gras; des glycérides ; le polyisobutène hydrogéné, des huiles hydrogénées concrètes à 25°C; des lanolines; des esters gras concrets à 25°C.

Ces corps gras peuvent en particulier être choisis de manière variée par l'homme du métier afin de préparer une composition ayant les propriétés souhaitées, par exemple en consistance ou en texture.

Ainsi, la phase grasse de l'émulsion selon l'invention peut être présente à une teneur comprise entre 10 % et 40 % en poids par rapport au poids total de la composition et de préférence comprise entre 20 % et 35 % en poids.

En outre, la composition selon l'invention peut comprendre au moins un agent stabilisant approprié pour une émulsion E/H à forte teneur en électrolyte. De tels stabilisants sont choisis notamment parmi les sels de métaux alcalins, par exemple le chlorure de sodium, les cires et leurs mélanges en toutes proportions.

Parmi les cires utiles comme stabilisant selon l'invention, on peut citer les cires animales, végétales, minérales et synthétiques telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; les cires de silicone. De préférence, on emploiera de la cire microcristalline.

La quantité d'agent stabilisant est avantageusement comprise entre 0 et 5 % en poids par rapport au poids total de la composition, de préférence comprise entre 0 et 3 % en poids.

Les compositions selon l'invention se présentent avantageusement sous forme de crème, de lait, de lotion, etc.

De façon connue, l'émulsion de l'invention peut contenir également des adjuvants habituels dans les domaines cosmétique et/ou dermatologique, tels que les conservateurs, les antioxydants, les agents complexants, les solvants, les parfums, les charges (par exemple un agent matifiant), les filtres, les bactéricides, les absorbeurs d'odeur, les matières colorantes, etc. Les quantités de ces différents adjuvants sont celles classiquement utilisées dans le domaine considéré, et par exemple de 0,01 à 5 % en poids du poids total de la composition. Ces adjuvants, selon leur nature, peuvent être introduits dans la phase grasse ou dans la phase aqueuse.

La composition selon l'invention peut également comprendre au moins un composé actif susceptible de provoquer une irritation cutanée.

La composition selon l'invention peut également être employée pour diminuer l'effet irritant d'au moins un composé actif administré séparément dans une composition cosmétique ou pharmaceutique, par voie topique (crème, lotion, gel, etc.) ou systémique (orale, rectale, parentérale). La composition selon l'invention peut être appliquée sur la peau simultanément à l'administration de l'actif susceptible d'irriter, ou de manière décalée dans le temps.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon et des extraits bactériens ou végétaux, notamment ceux d'Aloe Vera.

Comme actifs lipophiles, on peut utiliser le rétinol (vitamine A) et ses dérivés, le tocophérol (vitamine E) et ses dérivés, les acides gras essentiels, les céramides, les huiles essentielles.

Ces actifs peuvent être destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, les rétinoïdes notamment ceux décrits dans les demandes de brevet FR-A- 2 570 377, EP-A-199 636, EP-A-325 540, EP-A-402 072, le rétinal, la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes tels que l'amphotéricine B, les composés de la famille des allylamines, tels que la terbinafine, ou encore l'octopirox ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents antiviraux tels que l'acyclovir ;
- les agents kératolytiques tels que les acides alpha- et bêta-hydroxy-carboxyliques ou bêta-cétocarboxyliques, leurs sels, amides ou esters et plus particulièrement les alpha-hydroxyacides tels que l'acide glycolique, l'acide lactique, l'acide tartrique, l'acide malique, l'acide citrique, l'acide mandélique et de manière générale les acides de fruits, et les bêta-hydroxyacides comme l'acide salicylique et ses dérivés notamment alcoylés comme l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les antimétabolites ;
- les agents anti-chute des cheveux comme le minoxidil ;
- les antiseptiques.

La composition selon l'invention peut également comprendre comme actif au moins un agent kératolytique et/ou au moins un antagoniste de neuropeptide et/ou au moins un antagoniste de médiateur de l'inflammation, différents des sels métalliques hydrosolubles, notamment pour traiter les peaux sensibles.

La présente invention concerne donc également une composition sous forme d'émulsion E/H à forte teneur en électrolytes, comprenant au moins 2 % en poids par rapport au poids total de la composition d'au moins un sel métallique hydrosoluble tel que défini ci-dessus, au moins un composé actif choisi parmi les agents kératolytiques, les antagonistes de neuropeptide et les antagonistes de médiateur de l'inflammation, différents des sels métalliques hydrosolubles, au moins un gélifiant hydrophobe tel que défini ci-dessus et un système émulsionnant approprié tel que défini ci-dessus.

Comme agents kératolytiques utiles selon l'invention, on peut citer que les acides alpha- et bêta-hydroxy-carboxyliques ou bêta-cétocarboxyliques, leurs sels, amides ou esters et plus particulièrement les alpha-hydroxyacides tels que l'acide glycolique, l'acide lactique, l'acide tartrique, l'acide malique, l'acide citrique, l'acide mandélique et de manière générale les acides de fruits, et les bêta-hydroxyacides comme l'acide salicylique et ses dérivés notamment alcoylés comme l'acide n-octanoyl-5-salicylique. De manière avantageuse, ces agents kératolytiques peuvent être présents dans la composition selon l'invention en des quantités allant jusqu'à 10 % en poids par rapport au poids total de la composition, de préférence compris entre 0,1 et 5 % en poids.

Comme antagonistes de neuropeptides utiles dans l'invention, on peut citer les antagonistes de substance P et les antagonistes de CGRP et, comme antagonistes de médiateurs de l'inflammation, on peut citer les antagonistes d'histamine, d'interleukine 1 ou de TNFα. Ces antagonistes peuvent être présents à raison de 0,000001 à 10 % en poids du poids total de la composition et de préférence de 0,0001 à 5 %.

De façon avantageuse, on utilise de préférence des antagonistes réceptoriels de substance P, de CGRP et/ou d'interleukine 1.

Comme antagoniste de substance P utiles selon l'invention, on peut citer toute substance d'origine organique ou minérale, capable de produire une inhibition de la fixation réceptorielle de substance P ou une inhibition de la synthèse et/ou la libération de substance P par des fibres nerveuses sensitives.

L'antagoniste réceptoriel de substance P peut être notamment un peptide ou un dérivé non peptidique comportant un hétéroatome, et plus précisément un composé comportant un hétérocycle ou un hétéroatome lié directement ou indirectement à un cycle benzènique.

On peut utiliser par exemple comme peptide antagoniste de substance P réceptoriel le sendide et le spantide Il.

On peut également utiliser dans l'invention comme peptide ceux décrits dans les brevets et demandes de brevet US-A-4 472 305, US-A-4 839 465, EP-A-101 929, EP-A-333 174, EP-A-336 230, EP-A-394 989, EP-A-443 32, EP-A-498 069, EP-A-515 681, EP-A-517 589, WO-A-92/22569 et GB-A-2 216 529.

Les antagonistes réceptoriels de substance P non peptidiques utiles selon l'invention sont notamment des composés hétérocycliques notamment soufrés, azotés ou oxygénés ou des composés comprenant un atome d'azote lié directement ou indirectement à un cycle benzénique.

Comme composé hétérocyclique, on peut utiliser selon l'invention ceux décrits dans les demandes de brevet suivantes : EP-A-360 390, EP-A-429 366, EP-A-430 771, EP-A-499 313, EP-A-514 273, EP-A-514 274, EP-A-514 275, EP-A-514 276, EP-A-520 555, EP-A-528 495, EP-A-532 456, EP-A-545 478, EP-A-558 156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01 169, WO-A-93/01170, WO-A-93/06099, WO-A-93/09116 et WO-A-94/08997. En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle ou un dérivé d'isoindole.

Comme composés comportant un atome d'azote lié directement ou indirectement à un noyau benzénique, on peut citer ceux décrits dans les demandes de brevet EP-A-522 808 et WO-A-93/01165.

Comme antagoniste de CGRP utiles selon l'invention, on peut citer toute substance d'origine organique ou minérale capable de produire une inhibition de la fixation réceptorielle du CGRP ou de produire une inhibition de la synthèse et/ou de la libération de CGRP par les fibres nerveuses sensitives.

Pour qu'une substance soit reconnue comme un antagoniste de CGRP, elle doit répondre notamment à la caractéristique suivante de présenter une activité pharmacologique antagoniste du CGRP, c'est-à-dire induire une réponse pharmacologique cohérente notamment dans l'un des tests suivants:
- la substance antagoniste doit diminuer la vasodilatation induite par la capsaïcine et/ou
- la substance antagoniste doit provoquer une inhibition de la libération de CGRP par les fibres nerveuses sensitives et/ou
- la substance antagoniste doit provoquer une inhibition de la contraction du muscle lisse du canal déférent induite par le CGRP.

En outre, l'antagoniste de CGRP peut avoir une affinité pour les récepteurs au CGRP.

On peut utiliser dans l'invention par exemple comme antagoniste réceptoriel de CGRP, le CGRP 8-37, un anticorps anti-CGRP.

Lorsque la composition selon l'invention comprend un composé actif susceptible de provoquer une irritation cutanée choisi parmi les agents kératolytiques, les antagonistes de neuropeptide et les antagonistes de médiateur de l'inflammation, différents des sels métalliques hydrosolubles, la quantité de système émulsionnant sera appropriée pour obtenir une composition stable, selon les critères de stabilité définis ci-dessus. Là encore, on cherchera à employer la quantité minimum de système émulsionnant permettant d'obtenir un composition stable. Une quantité appropriée de système émulsionnant est donc une quantité nécessaire et suffisante pour obtenir une composition stable au moins 1 mois à 45°C. Elle est de préférence inférieure à 10 % en poids par rapport au poids total de la composition, plus préférentiellement inférieure à 8 % en poids.

Les exemples ci-après d'émulsions selon l'invention sont des exemples donnés à titre d'illustration et sans caractère limitatif. Les quantités y sont données en % en poids par rapport au poids total de la composition.

### Exemple 1 : Crème E/H

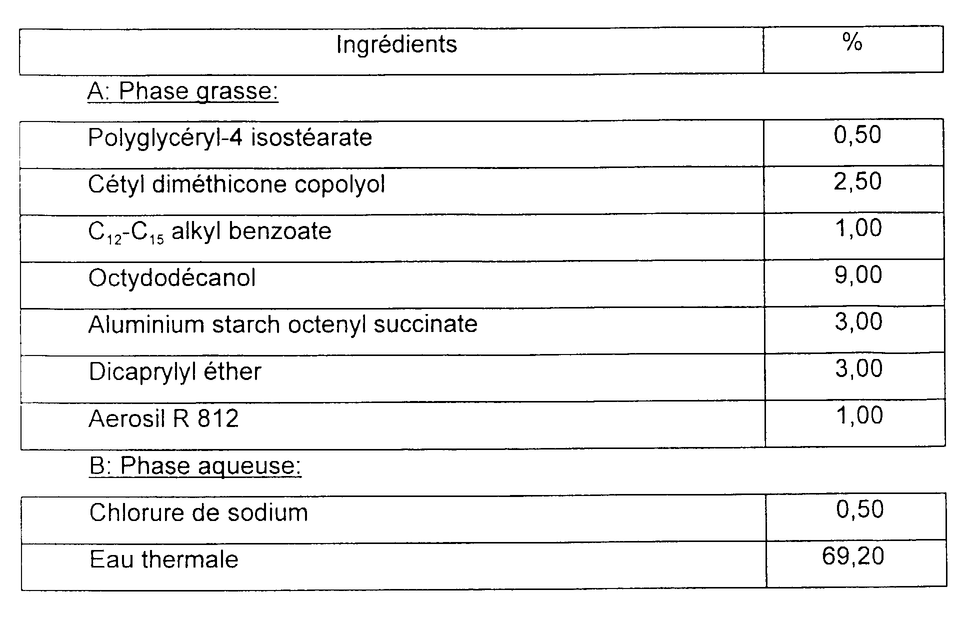

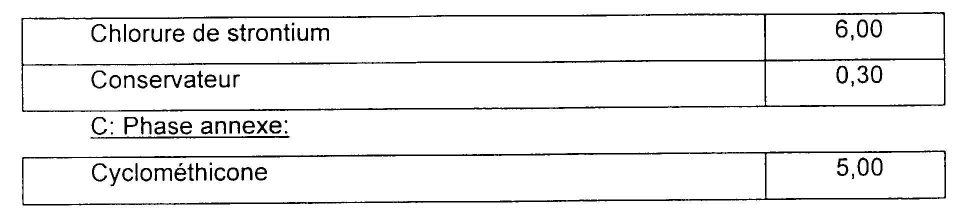

La crème E/H est préparée selon le mode opératoire suivant:
- on fait chauffer les phases A et B à 75°C séparément, puis on verse progressivement à 600 tr/mn la phase B dans la phase A;
- on laisse refroidir sous agitation à 600 tr/mn jusqu'à 40°C;
- on ajoute alors la phase C à 1500 tr/mn, puis on agite l'émulsion à 3000 tr/mn pendant 5 min.

On obtient une émulsion stable pendant au moins 1 mois à 45°C

### Exemple 2 : Crème E/H

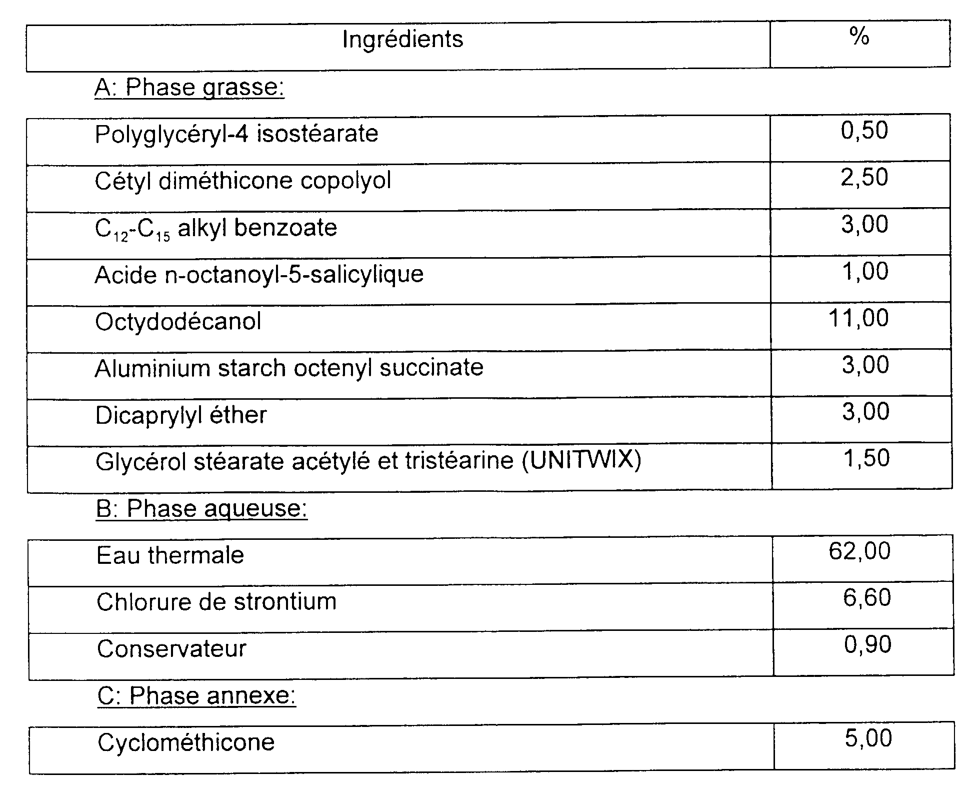

L'émulsion est préparée en suivant le mode opératoire de l'exemple 1. On obtient également une composition stable au moins 1 mois à 45°C.

### Exemple 3 : Crème E/H

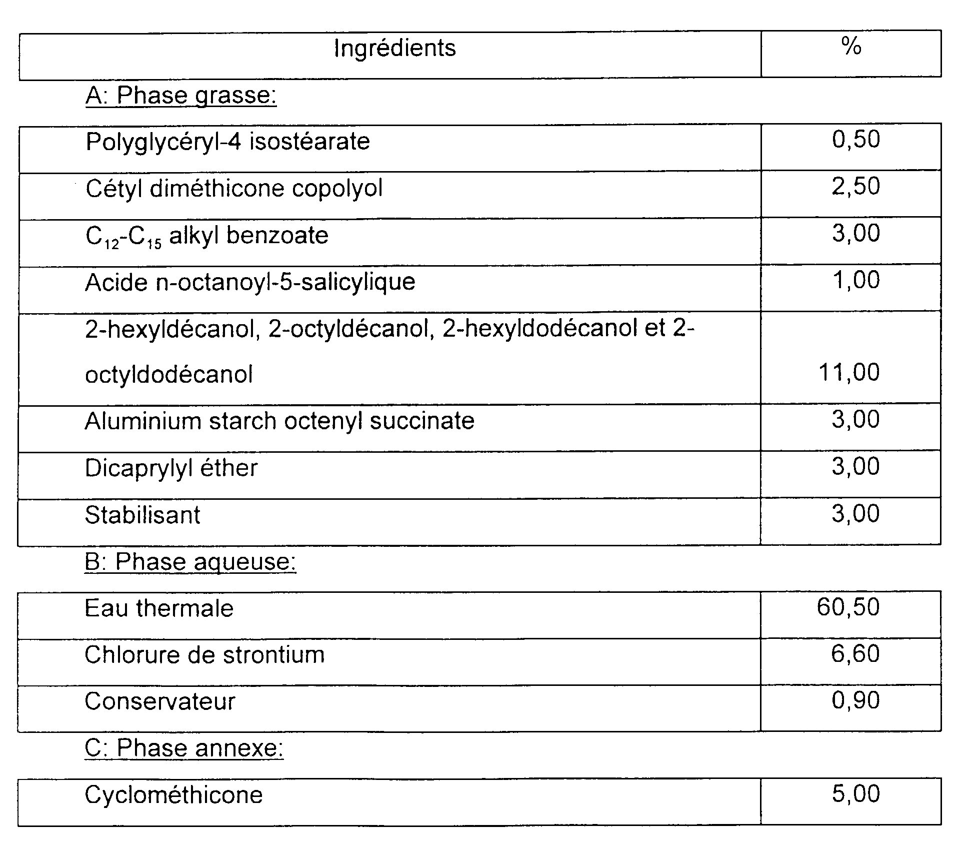

L'émulsion est préparée en suivant le mode opératoire de l'exemple 1. On obtient également une composition stable au moins 1 mois à 45°C.

Les émulsions E/H selon l'invention ont montré une meilleure tolérance pour les sels de métaux alcalino-terreux que les émulsions H/E correspondantes, pour les personnes atteintes de prurit.

## Revendications

1. Composition sous forme d'émulsion E/H à forte teneur en électrolytes, caractérisée en ce qu'elle comprend au moins 2 % en poids par rapport au poids total de la composition d'au moins un sel métallique hydrosoluble, au moins un gélifiant hydrophobe et moins de 5 % en poids par rapport au poids total de la composition d'un système émulsionnant approprié.

2. Composition selon la revendication 1, caractérisée en ce qu'elle comprend au moins 3 % en poids d'au moins un sel métallique hydrosoluble, de préférence au moins 5 % en poids.

3. Composition selon l'une des revendications 1 ou 2, caractérisée en ce que les sels métalliques hydrosolubles sont choisis parmi les sels hydrosolubles de métaux alcalins, de métaux alcalino-terreux, de métaux de transition et de métaux des groupes 13 et 14 de la classification périodique des éléments.

4. Composition selon l'une des revendications 1 à 3, caractérisée en ce que les sels métalliques hydrosolubles sont choisis parmi les sels de lithium, de strontium, de baryum, d'yttrium, de néodyme, de gadolinium, de manganèse et de zinc, de préférence de strontium.

5. Composition selon l'une des revendications 1 à 4, caractérisée en ce que les sels métalliques hydrosolubles sont choisis parmi les carbonates, bicarbonates, sulfates, glycérophosphates, borates, chlorures, nitrates, acétates, hydroxydes, persulfates, les sels d'α-hydroxyacides, les sels d'acides aminés ou les sels d'acides gras .

6. Composition selon l'une des revendications 1 à 5, caractérisée en ce que la quantité de sels métalliques hydrosolubles est comprise entre 2 % et 20 % en poids par rapport au poids total de la composition, de préférence comprise entre 5 % et 10 % en poids.

7. Composition selon l'une des revendications 1 à 6, caractérisée en ce que le système émulsionnant approprié est constitué par au moins un ester de glycérol et/ou au moins un polymère siliconé, appropriés pour la préparation d'émulsions E/H.

8. Composition selon la revendication 7, caractérisée en ce que l'ester de glycérol approprié pour la préparation d'une émulsion E/H est un ester de polyglycérol et d'acide gras, en particulier un ester de polyglycérol et d'acide (iso)stéarique, tels que les polyglycéryl-n isostéarates, n étant un entier compris entre 2 et 6.

9. Composition selon la revendication 7, caractérisée en ce que le polymère siliconé approprié pour la préparation d'une émulsion E/H est un polysiloxane, de préférence un copolymère de polysiloxane polyalkyle polyéther.

10. Composition selon la revendication 9, caractérisée en ce que le copolymère de polysiloxane polyalkyle polyéther est choisi parmi le cétyl diméthicone et le lauryl méthicone copolyol.

11. Composition selon l'une des revendications 7 à 10, caractérisée en ce que le système émulsionnant approprié est constitué par l'association d'au moins un polymère siliconé et d'au moins un ester de glycérol, le rapport pondéral ester de glycérol / silicone étant compris entre 1/1 et 1/10, plus préférentiellement d'environ 1/5.

12. Composition selon l'une des revendications 1 à 11, caractérisée en ce que la quantité de système émulsionnant approprié est comprise entre 2 % et 4 % en poids par rapport au poids total de la composition.

13. Composition selon l'une des revendications 1 à 11, caractérisée en ce que le gélifiant hydrophobe est choisi parmi les argiles modifiées, la silice hydrophobe, les dérivés d'esters de glycol et d'acide gras.

14. Composition selon la revendication précédente, caractérisée en ce que le gélifiant hydrophobe est un mélange de stéarate de glycol acétylé et de tri-ester de glycérol et d'acide gras.

15. Composition selon la revendication 13, caractérisée en ce que le gélifiant hydrophobe est une silice silylée ou diméthyl silylée.

16. Composition selon l'une des revendications 1 à 15, caractérisée en ce que la quantité de gélifiant hydrophobe est comprise entre 0,01 et 5 % en poids par rapport au poids total de la composition.

17. Composition selon l'une des revendications 1 à 16, caractérisée en ce que la phase aqueuse comprend de l'eau, de l'eau purifiée, une eau florale telle que l'eau de bleuet, ou une eau thermale ou minérale naturelle, et leurs mélanges en toutes proportions.

18. Composition selon l'une des revendications 1 à 17, caractérisée en ce que la phase aqueuse est présente à une teneur comprise entre 40 % et 80 % en poids par rapport au poids total de la composition, de préférence comprise entre 50 % et 70 % en poids.

19. Composition selon l'une des revendications 1 à 18, caractérisée en ce que la phase grasse est présente à une teneur comprise entre 10 % et 40 % en poids par rapport au poids total de la composition et de préférence comprise entre 20 % et 35 % en poids.

20. Composition selon l'une des revendications 1 à 19, caractérisée en ce qu'elle comprend en outre des composés actifs susceptibles de provoquer une irritation cutanée.

21. Produit de combinaison comprenant une composition selon l'une des revendications 1 à 19 et une composition cosmétique ou pharmaceutique comprenant au moins un composé actif susceptible de provoquer une irritation cutanée, pour une utilisation séparée, simultanée ou décalée dans le temps pour diminuer l'effet irritant du composé actif administré séparément.

22. Composition sous forme d'émulsion E/H à forte teneur en électrolytes, caractérisée en ce qu'elle comprend au moins 2 % en poids par rapport au poids total de la composition d'au moins un sel métallique hydrosoluble tel que défini dans les revendications 1 à 6, au moins un composé actif choisi parmi les agents kératolytiques, les antagonistes de neuropeptide et les antagonistes de médiateur de l'inflammation, différents des sels métalliques hydrosolubles, au moins un gélifiant hydrophobe approprié tel que défini dans les revendications 13 à 16 et un système émulsionnant approprié tel que défini dans les revendications 7 à 12.
